Europäisches Patentamt

European Patent Office

Office européen des brevets

(11) Publication number: **0 347 708**
**A1**

## EUROPEAN PATENT APPLICATION

(21) Application number: 89110632.0

(51) Int. Cl.⁴: **A61B 5/02** , **A61N 1/39**

(22) Date of filing: 12.06.89

(30) Priority: 16.06.88 US 207468

(43) Date of publication of application:
27.12.89 Bulletin 89/52

(84) Designated Contracting States:
BE CH DE ES FR GB IT LI NL SE

(71) Applicant: Cardiac Pacemakers, Inc.
4100 Hamline Avenue North
St. Paul Minnesota 55164-5798(US)

(72) Inventor: Shapland, J. Edward
4322 Rustic Place
Shoreview Minnesota 55126(US)
Inventor: Salo, Rodney W.
136 71st Way, N.E.
Fridley Minnesota 55432(US)
Inventor: Lincoln, William C.
11783 Narcissus Street
Minneapolis Minnesota 55433(US)

(74) Representative: Weber, Otto Ernst, Dipl.-Phys.
et al
Weber & Heim Hofbrunnstrasse 36
D-8000 München 71(DE)

(54) Hemodynamic tachyarrhythmia detection.

(57) A method is disclosed for measuring normal heart function over a predetermined time threshold. These measurements are compared to measured RV end diastolic pressure/volume, right atrial filling pressure, right atrial peak volume, and/or mean right ventricular or right atrial pressure/volume which, if exceeding the normal heart measurements for a predetermined time threshold (18,28) is indicative of abnormal heart function (20,30). This indication taken alone or in combination with a predetermined heart rate in excess of a normal heart rate for a predetermined time, results in application of a defibrillating shock (36) to the heart of the patient. RV and diastolic pressure/volume, right atrial filling pressure, right atrial peak volume, and/or mean right ventricular or right atrial pressure/volume are the most consistent and instantaneous reflections of abnormal heart conditions which trace systemic pressure/flow.

FIG. 4

## HEMODYNAMIC TACHYARRHYTHMIA DETECTION

### Field of the Invention

This invention relates to the detection and evaluation of the hemodynamic condition of a patient during tachycardia and fibrillation.

### Background of the Invention

Ventricular fibrillation (VF) and certain ventricular tachycardias (VT) are lethal cardiac arrhythmias (hereinafter sometimes collectively referred to as VF) for which the only known efficacious treatment is electrical countershock. A victim of VF or life-threatening VT outside of a hospital setting has little chance of survival, since treatment must take place within a few minutes of the onset of the episode.

Fortunately, new techniques and devices are being devised to help deal with these life-threatening conditions. Among these are computer techniques which aid in the identification of high-risk patients, anti-arrhythmic drugs which can prophylactically be administered to these patients, programs for widespread cardiopulmonary resuscitation training, and implantable devices which can automatically detect VF and VT, and deliver cardioverting countershocks.

"Cardioverting" or "cardioversion", as used herein, is intended to encompass the correction of a number of arrhythmic heart conditions, both lethal and non-lethal. Those arrhythmic heart conditions include atrial tachycardia, atrial flutter, atrial fibrillation, junctional rhythms, ventricular tachycardia, ventricular flutter, ventricular fibrillation, and any other non-pacemaking related arrhythmic condition which may be corrected by applying electrical shocks to the heart. Obviously then, "defibrillation" is included in the term cardioversion as a method of applying electrical shocks to the heart to defibrillate fibrillating atria or fibrillating ventricles.

Many of the known techniques, such as defibrillation by an automatic implantable defibrillator have been developed which are generally acceptable for detecting VF and discriminating between life-threatening arrhythmias and other cardiac malfunctions. Yet there is room for alternative methods and devices for detecting and discriminating VF from other non-fatal arrhythmias.

While it is not possible to predict with unerring exactness which patient suffering from coronary heart disease will be the victim of sudden death, several high-risk groups of patients can be recognized. For example, patients who have experienced myocardial infarction, even though they may be surviving in good health, run a substantial risk of dying suddenly, a risk several times greater than that associated with the general population. Further, if patients with myocardial infarction have a history of serious ventricular arrhythmias and/or of cardiac arrest, or if evidence of persistent myocardial irritability is present, it may be logically assumed that the risk of sudden death is increased substantially.

In Reissue Patent No. 27,757 to Mirowski, a method and means for automatically defibrillating a malfunctioning heart is described. The patent describes the continuous monitoring of the heart function so that when the heart function becomes abnormal, the malfunctioning heart is automatically shocked by a voltage of substantial size. Normal heart activity ensures that the shocking mechanism remains inert.

The defibrillator of the Mirowski patent includes a capacitor capable of storing electrical energy in an amount sufficient to depolarize the human heart. Upon discharge of this capacitor, a shock is delivered to the heart through two stimulating electrodes. One of these electrodes is positioned within the right ventricle, thereby forming the distal tip of an intracardiac catheter. The capacitor is associated with a sensing circuit connected to the proximal end of the intracardiac catheter and is adapted to respond to a signal recorded at the distal end of the catheter. The signal sensed by the catheter is related to a distinctive characteristic of ventricular tachycardia or ventricular fibrillation. This characteristic is the pulsatile pressure in the right ventricle. When this pressure falls below a given value, on the order of 10-15 mm Hg., the pulsatile pressure no longer is detected, and the heart is considered to be malfunctioning; therefore, the capacitor is discharged into the heart.

U.S. Patent No. 4,202,340 to Langer et al. discloses a method and an apparatus for monitoring heart activity, for indicating abnormalities in such activity, and for taking corrective measures to return an arrhythmic heart to normal sinus rhythm. Cardiac electrodes are used to sense pulsatile impedance changes, which are absent in the presence of fibrillation. A VF detector in the form of an impedance sensor is used to measure impedance between cardiac electrodes. It has been found that the impedance due to cardiac contractions is related to stroke volume. According to this patent, during fibrillation, stroke volume falls substantially to zero, and a drastic drop in pulsatile impedance, which can be detected in the right ventricle, is used as an indicator of VF.

## Summary of the Invention

Many ventricular tachycardias (VT) are hemodynamically unstable, with the patient becoming syncopal and/or completely losing consciousness. However, other VT patients tolerate the arrhythmias without severe hemodynamic compromise. Rate of the VT alone cannot identify or predict which patients will have hemodynamic problems or the extent of the problems. Therefore, if a method were available to monitor hemodynamics during a VT and such a method could be included within an automatic implantable cardioverter/defibrillator (AICD), then the delivered therapy would be determined by the hemodynamic condition of the patient. That is, anti-tachy pacing termination (which may require longer conversion time) could be employed for patients with adequate hemodynamics and blood flow, or defibrillation (high energy) shocks could be employed for patients with hemodynamic collapse (which requires immediate conversion).

Problems encountered with this concept involve development of chronic sensor technology, and the identification of which hemodynamic parameter(s) to monitor. Recent advances in sensor development may provide pressure sensors or ventricular volume sensors (based on impedance techniques). However, the question of which parameters to monitor has remained. The preferred hemodynamic parameter to monitor would be blood flow from the left ventricle (LV) or systemic arterial or LV pressure. However, it is not desirable to chronically implant sensors in the LV or major arteries.

The invention relates to a method and apparatus for sensing certain parameters in the right heart or venous system that closely track hemodynamic changes in the left heart and hence arterial blood flow. Based on studies conducted to arrive at the present invention, the hemodynamics [right ventricular (RV) and systemic pressures and RV impedance volume] were monitored, and it was discovered that several parameters measured in the RV or right atrium accurately and quickly reflect LV blood flow. More particularly, it was learned that by monitoring right heart end-diastolic pressure or volume, alone or in combination with other right heart parameters, an implanted AICD would have a clear indicator of the patient's overall hemodynamic condition and could give the most appropriate form of therapy (pace termination or high energy shocks) accurately and without delay.

Therefore, the key points in assessing general hemodynamics during a tachycardia is the use of specific pressure and volume parameters from the right ventricle (RV) or right atrium. Previous systems have been confined to the right ventricular pulsatile pressure, as in the patent to Mirowski, and its correlation to VT and VF. By the present invention, the mean right ventricular or right atrial pressure or volume, right atrial filling pressure, or right atrial peak volume, and/or right ventricular end diastolic volume or pressure is examined, and yields a better correlation to overall patient hemodynamics and arterial pressure than in the patents noted above.

One embodiment of the invention includes the placement of a chronic pressure transducer in the right ventricle or right atrium. In this position, right ventricular or right atrial end diastolic and mean pressures are monitored. A second embodiment involves placement of an impedance sensor or other volume sensor in the right atrium or right ventricle. From either of these two embodiments, monitoring of mean pressure or end diastolic pressure/volume is used to detect hemodynamically unstable ventricular tachycardias.

These measurements are incorporated into an algorithm, which not only monitors mean and/or diastolic pressure/volumes, but also rate. By considering these parameters along with the heart rate of a patient, the decision can be made whether or not the patient is suffering from severe hemodynamically compromising ventricular tachycardias or relatively well-tolerated ventricular tachyarrhythmias. The algorithms can also be used to direct automatic · implantable cardioverter/defibrillator (AICD) to treat the unstable arrhythmias more aggressively with high energy shocks or hemodynamically stable arrhythmias less aggressively with anti-tachycardia pacing or perhaps low energy shocks.

It is an object of the present invention to monitor specific right heart volume or pressure parameters, specifically end diastolic pressure or volume, atrial filling pressure or peak volume, or mean pressure or volume, to determine the hemodynamics of a patient.

It is another object of the present invention to monitor end diastolic pressure or volume or mean pressure or volume in the right ventricle to accurately reflect left ventricular blood flow.

It is yet another object of the present invention to monitor end diastolic pressure or volume or mean pressure or volume in the right ventricle to accurately reflect left ventricular blood flow to determine if a patient is suffering from severe hemodynamically compromising ventricular tachycardias or relatively well-tolerated ventricular tachyarrhythmias.

These and other objects of the present invention, as well as many of the attendant advantages thereof, will become more readily apparent when reference is made to the following description, taken in conjunction with the accompanying drawings.

## Brief Description of the Drawings

Figure 1 is a side-by-side comparison of charts for normal and abnormal systemic pressure, RV impedance, RV pressure, right atrial (RA) electrocardiogram, global RV electrocardiogram, and local RV electrocardiogram.

Figure 2 is a graph representing pressure/volume change during ventricular tachycardia.

Figure 3 is a flowchart of a discrimination algorithm.

Figure 4 is a block diagram of a sensing circuitry and shock mechanism.

## Detailed Description of the Preferred Embodiments

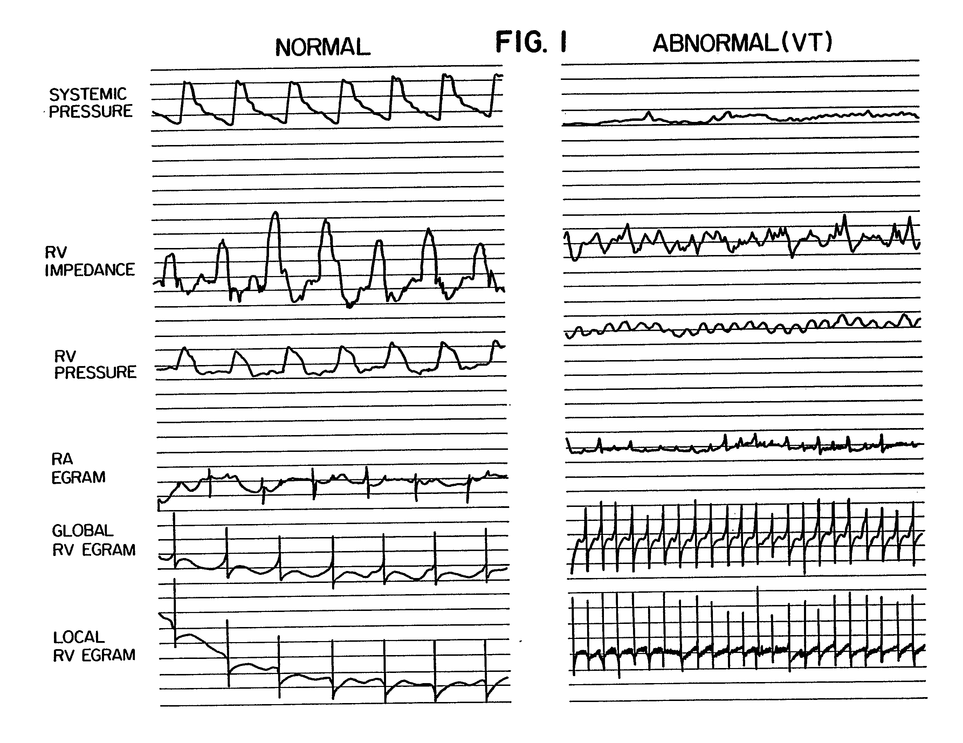

With reference to Figure 1, charts for normal and abnormal (during ventricular tachycardia) conditions of the heart are shown for six different parameters. This comparison shows the extreme divergence between that of a normal and abnormal condition of the heart.

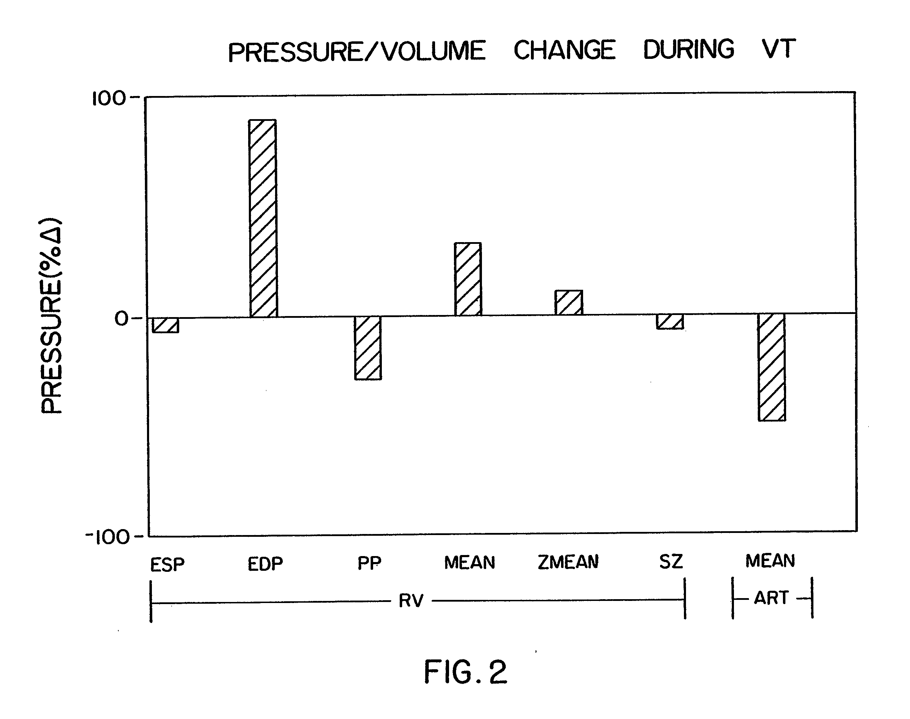

To demonstrate the significance in a change of percentage in pressure/volume during ventricular tachycardia, test results were compiled to form the graph of Figure 2. The changes of percentage for mean arterial pressure at the right-hand side of the graph is indicative of a drastic change in pressure/volume for 10 heartbeats during VT as compared to 10 heartbeats under normal sinus rhythm. The change from 10 beats of sinus rhythm to 10 beats of VT is illustrated for six parameters.

It is noted that the end systolic pressure (ESP) changes little in percentage, whereas the end diastolic pressure (EDP) gives the greatest positive change in percentage during ventricular tachycardia. The change in percentage in pressure/volume for EDP is almost 100 percent. The pulsatile pressure (PP) and mean right ventricular pressure (mean) change in percentage from a normal condition to VT in an almost equal amount, albeit in different directions, with the PP producing a decrease, whereas the mean exhibits an increase. Of smaller amplitude, although equally consistent, is the change in percentage of the mean impedance (Z mean). Relatively erratic changes were noted in pulsatile impedance (SZ) analogous to stroke volume, with the average change being quite small.

This graph supports the discovery that during ventricular tachycardia, the pulsatile pressure and pulsatile impedance measured in the right ventricle under prior practice as an indicator of VT actually severely lagged the instantaneous occurrence of VT. It was found that it was possible for the left ventricle to be in VT for 10-15 seconds before the right ventricle would demonstrate pulsatile parameters indicative of VT. Since the average blood volume in the right ventricle goes up immediately when the pumping activity is compromised, the end diastolic pressure or volume was found to provide instantaneous evidence of a pathologic VT of the heart.

To provide instantaneous feedback on the condition of the heart, the end diastolic pressure of the right ventricle may be measured with a pressure sensor and a peak detector. In addition, the mean pressure of the right ventricle has been found to provide an accurate, instantaneous indication of abnormal heart conditions since the volume in the right ventricle increases during a hemodynamically comprising VT, the mean pressure increases. A pressure sensor is used to monitor the mean pressure in the right ventricle or an impedance sensor may be used to measure the volume. The mean pressure or volume is obtained by passing the raw pressure or volume signal through a low-pass filter.

In addition, as the mean right ventricular pressure and volume increase during a hemodynamically compromising VT, the filling pressure, reflected in the right atrial pressure, as well as the peak right atrial volume, must also increase. Thus, the atrial pressure measured with a pressure transducer in the right atrium or the right atrial volume measured by impedance in the atrium must be used to characterize VTs.

The noted parameters providing instantaneous indication of an abnormal heart condition may also be used as a secondary indicator in combination with a rate detector used as a primary detector of abnormal heart function. If the rate of the heart increases beyond a predetermined threshold, indicative of abnormal heart conditions, then the volume or pressure parameters of the right ventricle or atrial are checked; and, if they indicate a sufficient increase over previously stored values, a trigger mechanism provides a shock to the heart.

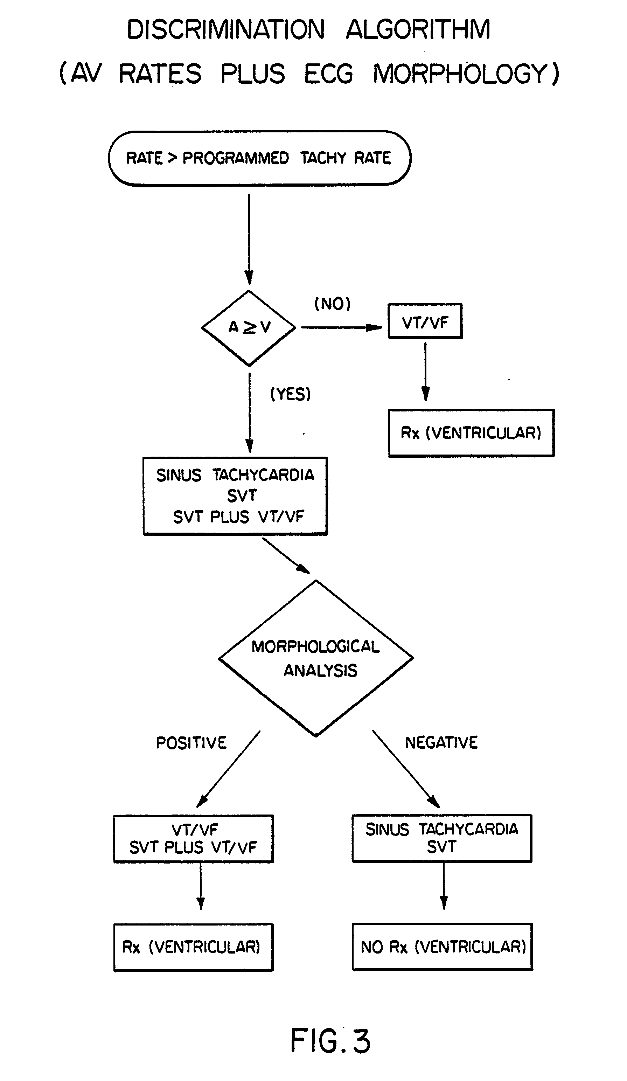

In Figure 3, the heart rate is first compared against a programmed tachycardia rate. If the atrial rate is less than the ventricular rate, ventricular tachycardia/ventricular fibrillation is considered to be present, and a high energy defibrillating shock is delivered to the heart.

However, if the atrial rate is greater than or equal to the ventricular rate, this is considered to be indicative of a sinus tachycardia, supra ventricular tachycardia (SVT), or SVT plus VT/VF. Under this scenario, a morphological analysis of the heart is conducted to measure the change in end diastolic pressure/volume or mean pressure/volume; and, if these parameters are compared to previously stored volumes and found to have increased sufficiently, this is considered to be indicative of VT/VF or SVT plus VT/VF, and a high energy defibrillating shock is delivered to the heart.

However, in contrast, if the end diastolic pressure or volume, or mean pressure or volume are found to be unchanged or to have decreased, this is considered to be indicative of sinus tachycardia, SVT, or a benign VT, and no shock is delivered to the heart.

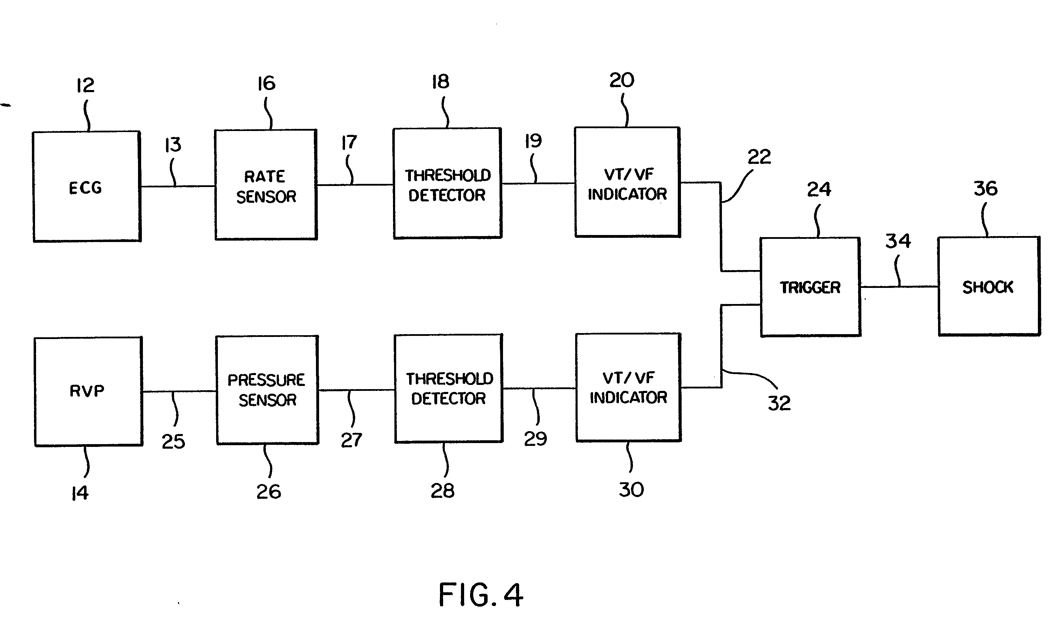

As illustrated in Figure 4, the heart of a patient produces measurable ECG signals 12 and right ventricular pressure (RVP) signals 14. A rate sensor 16 measures the ECG signals 12 generated across line 13 and compares these signals by transmission through line 17 to a threshold detector 18. If the ECG signals 12 exceed a predetermined threshold stored in the threshold detector 18, a signal is generated across line 19 to VT/VF indicator 20, which sends a signal across line 22 to trigger 24. Meanwhile, RVP signals 14 are detected across line 25 by pressure sensor 26. If the signals detected and transmitted across line 27 to the threshold detector 28 exceed previous stored values by a threshold value stored in threshold detector 28, a signal is generated across line 29 to VT/VF indicator 30 and along line 32 to trigger mechanism 24. Only in the presence of signals from both VT/VF indicators 20 and 30 will trigger mechanism 24 send a signal across 34 to deliver a shock 36 to the heart.

It is the intent that the present invention not be limited to the above, but be limited only as defined in the appended claims.

## Claims

1. A method of electronically monitoring the operation of a heart for immediate detection of abnormalities in cardiac function such as life-threatening arrhythmias, said method comprising: measuring mean right heart pressure over a predetermined time period, and comparing the measured mean right heart pressure against subsequently measured mean right heart pressure for a subsequent predetermined time period and when the subsequently measured mean right heart pressure exceeds said first measured mean right heart pressure by a predetermined threshold, an electrical signal is generated representative of an abnormal functioning of the heart.

2. The method of claim 1, wherein said measuring of mean right heart pressure is performed simultaneously with measuring of current heart rate so that when the measured current heart rate exceeds a predetermined baseline heart rate for a predetermined time period and said subsequently measured mean right heart pressure exceeds said first measured mean right heart pressure, said electrical signal triggers a mechanism to deliver a defibrillating shock to the heart.

3. A method of electronically monitoring the operation of a heart for immediate detection of abnormalities in cardiac function such as life-threatening arrhythmias, said method comprising: measuring mean right heart volume over a predetermined time period, and comparing the measured mean right heart volume against subsequently measured mean right heart volume for a subsequent predetermined time period and when the subsequently measured mean right heart volume exceeds said first measured mean right heart volume by a predetermined threshold, an electrical signal is generated representative of an abnormal functioning of the heart.

4. The method of claim 3, wherein said measuring of mean right heart volume is performed simultaneously with measuring of current heart rate so that when the measured current heart rate exceeds a predetermined baseline heart rate for a predetermined time period and said subsequently measured mean right heart volume exceeds said first measured mean right heart volume, said electrical signal triggers a mechanism to deliver a defibrillating shock to the heart.

5. A method of electronically monitoring the operation of a heart for immediate detection of abnormalities in cardiac function such as life-threatening arrhythmias, said method comprising: measuring right ventricular end diastolic pressure over a predetermined time period, and comparing the measured right heart end diastolic pressure against subsequently measured right heart end diastolic pressure for a subsequent predetermined time period and when the subsequently measured right heart end diastolic pressure exceeds said first measured right heart end diastolic pressure by a predetermined threshold, an electrical signal is generated representative of an abnormal functioning of the heart.

6. The method of claim 5, wherein said measuring of right ventricular end diastolic pressure is performed simultaneously with measuring of current heart rate so that when the measured current heart rate exceeds a predetermined baseline heart rate for a predetermined time period and said subsequently measured right ventricular diastolic pressure exceeds said first measured right ventricular end diastolic pressure, said electrical signal triggers a mechanism to deliver a defibrillating shock to the heart.

7. A method of electronically monitoring the operation of a heart for immediate detection of abnormalities in cardiac function such as life-threatening arrhythmias, said method comprising: measuring right ventricular end diastolic volume over a predetermined time period, and comparing the measured right heart end diastolic volume against subsequently measured right heart end diastolic volume for a subsequent predetermined time period and when the subsequently measured right heart end diastolic volume exceeds said first measured right heart end diastolic volume by a predetermined threshold, an electrical signal is generated representative of an abnormal functioning of the heart.

8. The method of claim 7, wherein said measuring of right ventricular end diastolic volume is performed simultaneously with measuring of current heart rate so that when the measured current heart rate exceeds a predetermined baseline heart rate for a predetermined time period and said subsequently measured right ventricular end diastolic volume exceeds said first measured right ventricular end diastolic volume, said electrical signal triggers a mechanism to deliver a defibrillating shock to the heart.

9. A method of electronically monitoring the operation of a heart for immediate detection of abnormalities in cardiac function such as life-threatening arrhythmias, said method comprising:
measuring right atrial filling pressure over a predetermined time period, and
comparing the measured right atrial filling pressure against subsequently measured right atrial filling pressure for a subsequent predetermined time period and when the subsequently measured right atrial filling pressure exceeds said first measured right atrial filling pressure by a predetermined threshold, an electrical signal is generated representative of an abnormal functioning of the heart.

10. The method of claim 9, wherein said measuring of right atrial filling pressure is performed simultaneously with measuring of current heart rate so that when the measured current heart rate exceeds a predetermined baseline heart rate for a predetermined time period and said subsequently measured right atrial filling pressure exceeds said first measured right atrial filling pressure, said electrical signal triggers a mechanism to deliver a defibrillating shock to the heart.

11. A method of electronically monitoring the operation of a heart for immediate detection of abnormalities in cardiac function such as life-threatening arrhythmias, said method comprising:
measuring right atrial peak volume over a predetermined time period, and
comparing the measured right atrial peak volume against subsequently measured right atrial peak volume for a subsequent predetermined time period and when the subsequently measured right atrial peak volume exceeds said first measured right atrial peak volume by a predetermined threshold, an electrical signal is generated representative of an abnormal functioning of the heart.

12. The method of claim 11, wherein said measuring of right atrial peak volume is performed simultaneously with measuring of current heart rate so that when the measured current heart rate exceeds a predetermined baseline heart rate for a predetermined time period and said subsequently measured right atrial peak volume exceeds said first measured right atrial peak volume, said electrical signal triggers a mechanism to deliver a defibrillating shock to the heart.

13. A method of electrically monitoring the operation of a heart for immediate detection of abnormalities in cardiac function, said method comprising:
sensing at least one cardiac parameter and transmitting said at least one cardiac parameter to an implanted automatic implantable cardioverter/defibrillator (AICD),
simultaneously detecting a heart rate of the heart,
comparing the detected heart rate against a predetermined threshold and, when the detected heart rate exceeds said predetermined threshold, comparing the transmitted electrical signals against a second predetermined threshold, and generating and delivering a high energy defibrillating shock to the heart from the AICD when said sensed cardiac parameters exceed said second predetermined threshold and providing a different course of treatment to the heart when said sensed cardiac parameters are less than said second predetermined threshold.

14. The method of claim 13, wherein said at least one parameter is mean right heart pressure.

15. The method of claim 13, wherein said at least one parameter is right heart end diastolic pressure.

16. The method of claim 14, wherein another of said at least one parameter is right heart end diastolic pressure.

17. The method of claim 13, wherein said at least one parameter is mean right heart volume.

18. The method of claim 13, wherein said at least one parameter is right heart end diastolic volume.

FIG. 1

NORMAL — ABNORMAL(VT)

SYSTEMIC PRESSURE

RV IMPEDANCE

RV PRESSURE

RA EGRAM

GLOBAL RV EGRAM

LOCAL RV EGRAM

EP 0 347 708 A1

PRESSURE/VOLUME CHANGE DURING VT

FIG. 2

EP 0 347 708 A1

# DISCRIMINATION ALGORITHM

# (AV RATES PLUS ECG MORPHOLOGY)

```
        ┌──────────────────────────────────┐
        │  RATE > PROGRAMMED  TACHY  RATE   │
        └──────────────────────────────────┘
                        │
                        ▼
                     ◇ A ≥ V ◇ ──(NO)──▶ ┌──────────┐
                        │                  │  VT/VF   │
                      (YES)                └──────────┘
                        │                        │
                        ▼                        ▼
        ┌──────────────────────────┐     ┌──────────────────┐
        │    SINUS TACHYCARDIA      │     │ Rx (VENTRICULAR) │
        │          SVT             │     └──────────────────┘
        │     SVT PLUS VT/VF        │
        └──────────────────────────┘
                        │
                        ▼
                ◇ MORPHOLOGICAL
                   ANALYSIS   ◇
        POSITIVE ╱         ╲ NEGATIVE
                ▼           ▼
┌──────────────────────┐   ┌──────────────────────┐
│       VT/VF          │   │   SINUS TACHYCARDIA   │
│   SVT PLUS VT/VF     │   │         SVT          │
└──────────────────────┘   └──────────────────────┘
            │                          │
            ▼                          ▼
┌──────────────────┐       ┌──────────────────────┐
│ Rx (VENTRICULAR) │       │  NO Rx (VENTRICULAR)  │
└──────────────────┘       └──────────────────────┘
```

# FIG.3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 074 126 (PURDUE RESEARCH FOUNDATION) <br> * abstract @ page 6, line 21 - page 8, line 31; figures 1-4 * | 3, 7,13, 17,18 | A 61 B 5/02 <br> A 61 N 1/39 |
| Y | US-A-4 625 730 (G.H.FOUNTAIN ET AL.) <br> * abstract @ column 4, line 8 - column 5, line 30; figures 1-4 * | 5-8, 13,15, 18 | |
| Y | US-A-4 730 619 (G.KONING ET AL.) <br> * the whole document * | 5-8, 13,15, 18 | |
| A | US-A-4 137 910 (D.H.MURPHY) <br> * abstract * | 13 | |
| P,X | US-A-4 774 950 (T.J.COHEN) <br> * the whole document * | 1, 2, 5,6, 9, 10,13-16 | |
| | ----- | | TECHNICAL FIELDS SEARCHED (Int. Cl.4) |
| | | | A 61 B <br> A 61 N |

The present search report has been drawn up for all claims

| Place of search | Date of completion of the search | Examiner |
|---|---|---|
| THE HAGUE | 19-09-1989 | HUNT,B.W. |